# EUROPEAN PATENT APPLICATION

(11) **EP 1 681 274 A2**
(43) Date of publication of application: **19.07.2006**
(21) Application number: 06075081.7
(22) Date of filing: 12.01.2006
(51) Int. Cl.: C02F 11/04, C12M 1/18

(54) **Method and device for carrying out a fermentation process in a reactor.**

(30) Priority: 17.01.2005 NL 1028048; 17.01.2005 NL 1028047
(71) Applicant: Orgaworld B.V., 5405 AC Uden (NL)
(72) Inventor: Kaskens, Henricus Joseph Marie, 5492 CD ST.-Oedenrode (NL); Van den Boomen, Henricus Nicolaas Cornelis, 5421 CN Gemert (NL)
(74) Representative: Van kan, Johan Joseph Hubert

(57) **Abstract**

A device (1) and a method for carrying out a fermentation process in a reactor (2), which reactor (2) comprises a liquid-permeable floor (3), two substantially parallel side walls (4) extending perpendicularly to the floor (3), a rear wall (13), and a ceiling part (5) disposed opposite the floor (3), which joins said side walls (4) and said rear wall (13), which device (1) further comprises a pipe (6) for discharging liquid from the reactor (2), which pipe (6) is disposed underneath the floor (3), and which device (1) further comprises means (7) for moisturising the material to be fermented that is present in the reactor (2) as well as means (8) for providing access to the reactor (2) for the purpose of supplying and discharging material (9) to be fermented and fermented material (9), respectively, and which device (1) furthermore comprises a pipe (14) for discharging biogas from the reactor (2).

## Description

The present invention relates to a device for carrying out a fermentation process in a reactor, which reactor comprises a liquid-permeable floor, two substantially parallel side walls extending perpendicularly to the floor, a rear wall, and a ceiling part disposed opposite the floor, which joins said side walls and said rear wall, which device further comprises a pipe for discharging liquid from the reactor, which pipe is disposed underneath the floor, and which device further comprises means for moisturising the material to be fermented that is present in the reactor as well as means for providing access to the reactor for the purpose of supplying and discharging material to be fermented and fermented material, respectively, and which device furthermore comprises a pipe for discharging biogas from the reactor.

Such a device is known, for example from Dutch patent publication NL 9401154, wherein waste from the agricultural industry and household waste (such as kitchen and garden waste) are mixed with a liquid material, in particular an anaerobic slurry, and the temperature of the mixture is raised to a value between 25 and 70 °C. A floating layer is formed, in which hydrolysis and acidification of solid organic material take place. The biogas formed in the methanogenous zone flows upwards and bubbles up through the floating layer, in which floating layer hydrolysis and an acidification of solid organic material take place. Said upward bubbling of the biogas being formed on the one hand contributes toward the flotation process, whilst on the other hand the floating layer is mixed and broken open. To prevent accumulation of acidification products in the floating layer, liquid must be removed from the methanogenous zone, which liquid is preferably sprayed over the floating layer, and acidic fermentation products are removed from the floating layer through percolation, which fermentation products are carried to the methanogenous zone.

When such fermentation of solid waste is carried out in a batchwise process in an existing device comprising a closed reactor, which reactor comprises a liquid-permeable floor, formation of a floating layer does not take place. A solid mass is present through which water percolates. The water is removed from the reactor through the liquid-permeable floor, after which the water can be used for moisturising the solid mass. Such a moisturised solid mass may exhibit specific flow characteristics if the percolation of water is not optimal, however, water layers may form in the solid mass. The moisturised solid mass may become too thinly liquid, so that the mass will behave as a liquid rather than as solid matter. This may cause the material to be fermented to shift. As a result of said shifting of a layer of the fermenting material, the material may exert a large pressure on the closing means of the reactor, thus forcing them open.

Such a situation will cause problems in particular when large reactors are used, in which considerable amounts of material are fermented. When said shifted layer of material presses against the doors or other closing means of the closed reactor with great force, a force will be developed such that the closing means are forced open, which may have serious consequences. When said forcing open of the closing means does not take place, a major pressure buildup may still take place on the closing means, however, as a result of which it will be difficult to remove the fermented material from the reactor. Part of the water in the mass to be fermented will be extracted from the mass through the liquid-permeable floor, but in the case of a non-optimum percolation this will not suffice, so that the risk of shifting of part of the material is considered to be great.

In the manufacturing industry, liquid waste is produced on a large scale, which waste is processed in particular in waste water purification plants. Carbon dioxide (CO₂) is formed during the decomposition of hydrocarbons, which significantly contributes to the greenhouse effect. The present inventors have found that such waste, in addition to solid waste products, can also be fermented, in which process the hydrocarbons that are present are converted into methane gas (CH₄), among other substances, which is used for generating the so-called green current. The fermentation process takes place in an airtight reactor, in which a mixture of CO₂ and CH₄ is formed. Such a gas mixture may cause an explosion, i.e. a very great heat development and an enormous increase of the gas volume, if there is an ignition source and a sufficient amount of air. Such a dangerous situation can occur in particular when the reactor is opened, viz. when oxygen can enter the reactor, so that an explosive gas mixture can be formed. This is one of the reasons why fermentation is always carried out in an airtight reactor. The second reason is that oxygen from the air is toxic to the methane bacteria that are frequently used in fermentation processes.

Generally, three phases can be distinguished in every usual fermentation process, viz. a first phase comprising the filling of a reactor with the material to be fermented and the graft material (containing microorganisms that carry out the fermentation) and the starting of the fermentation process. A second phase comprises the process of fermentation and a third phase comprises the termination of the fermentation process and the emptying of the reactor. Normally, oxygen is present in the reactor in the first phase before the reactor is filled. When the fermentation process is started, methane is formed by the microorganisms present in the graft material, whilst oxygen is still present in the reactor. In principle, no oxygen is present in the reactor any more in the second phase. In the third phase, oxygen (in the form of air) will enter the reactor when it is opened for being emptied. At that moment, methane is still present in the reactor as well. In particular in a batchwise reactor, which is opened after every charge, oxygen will regularly be present in the reactor, in contrast to a continuous reactor, to which new material to be fermented is supplied continuously without oxygen being present.

It is an object of the present invention to provide a device for carrying out a fermentation process in a reactor, wherein the problems as regards the shifting of part of the material to be fermented and the risk of the reactor door being blocked and/or being forced open are minimised.

Another object of the present invention is to provide a device for carrying out a fermentation process in a reactor, wherein the fermented material can easily be removed from the reactor.

Yet another object of the present invention is to provide a device for carrying out a fermentation process in a reactor, which device is capable of fermenting a large amount of material in one go.

Another object of the present invention is to provide a method and a device for carrying out a fermentation process in a reactor, wherein the risks of explosion of the gas mixture that is present in the reactor are minimised.

Another object of the present invention is to provide a method and a device for carrying out a fermentation process in a reactor, wherein the biogas that is formed in the reactor is usefully and sustainable reused.

One or more of the above objects are accomplished by means of a device as referred to in the introduction, which is characterized in that the reactor comprises a retaining wall, which retaining wall is disposed between the means for providing access to the reactor and the material to be fermented that is present on the floor, wherein the retaining wall is supported on the floor, being enclosed between the two side walls, and wherein the reactor is provided with a pipe for supplying a flushing gas to the reactor.

Using the device according to the present invention, a layer of material that may have shifted will be stopped by the retaining wall that is installed in the reactor, so that no pressure can be exerted on the means for closing the reactor. Furthermore, the methane gas-containing gas mixture that is present in the reactor is displaced by a flushing gas, so that the risk of explosion is considered to be practically zero. An inert gas that cannot form an explosive mixture with the methane gas that is present in the reactor, for example nitrogen, carbon dioxide and carbon monoxide, or mixtures thereof, can be used as the flushing gas.

In a second embodiment, the present invention furthermore relates to a device for carrying out a fermentation process in a reactor, which reactor comprises a liquid-permeable floor, two substantially parallel side walls extending perpendicularly to the floor, a rear wall, and a ceiling part disposed opposite the floor, which joins said side walls and said rear wall, which device further comprises a pipe for discharging liquid from the reactor, which pipe is disposed underneath the floor, and which device furthermore comprises means for moisturising the material to be fermented that is present in the reactor as well as means for providing access to the reactor for the purpose of supplying and discharging material to be fermented and fermented material, respectively, wherein the reactor comprises a retaining wall, which retaining wall is disposed between the means for providing access to the reactor and the material to be fermented that is present on the floor, wherein the retaining wall is supported on the floor, being enclosed between the two side walls.

Also when this embodiment of the device according to the present invention is used, a layer of material that may have shifted will be stopped by the retaining wall that is mounted in the reactor, so that there is no risk of a pressure being exerted on the means for closing the reactor.

The retaining wall that is used in one or more embodiments of the present invention is preferably built up of individual wall elements to be placed one on top of the other. Examples of such individual wall elements are wooden beams, wooden or plastic plates and the like.

Preferably, one or more passages through which liquid can flow will be present between the individual wall elements of the retaining wall. The advantage of this is that the liquid can drain away, so that the pressure behind the retaining wall will decrease. As a result, it will be easier to remove the retaining wall from the reactor after termination of the fermentation process.

In another embodiment, the liquid-permeable floor extends as far as the means for providing access to the reactor. That is, a liquid-permeable floor is present also between the retaining wall and the means for providing access to the reactor, such as the doors of the reactor, which liquid-permeable floor is also present under the material to be fermented. As a result of the presence of the liquid-permeable floor between the retaining wall and the means for providing access to the reactor, the liquid that flows through the retaining wall can be discharged, so that no pressure buildup will take place against the means for providing access to the reactor, so that the reactor can be opened in a safe manner after termination of the fermentation process and it is possible to enter the device.

The height of the retaining wall is not critical, any desired height may be used, depending on the amount of material to be fermented that is present in the reactor. In particular, the retaining wall will have a height greater than that of the material to be fermented. After the retaining wall has been installed in the reactor, the means for providing access to the reactor can be closed, thus creating a fully closed space.

During the fermentation process, the material to be fermented will be moisturized, in particular by means mounted in the ceiling part. It is preferable, however, to discharge the liquid that is produced during the fermentation process from the reactor via a pipe and use said liquid for moisturising the material to be fermented. To achieve this, the pipe can be connected to the aforesaid means, for example via a pump (possibly in combination with a purification plant).

A first effect achieved with the present invention is that the closing means of the reactor are prevented from being blocked and/or forced open by a shifted layer of fermented material. The use of the present invention comprising a retaining wall ensures that no forces will be exerted on the doors but that the forces are partially diverted and to be exerted on the retaining wall. In this way filling and emptying of the reactor can take place under safe conditions.

In addition to that the present invention enables large-scale fermentation. Normally, the use of such high-capacity devices would not be possible, since the pressure exerted on the closing means of the reactor would be too high. Since large amounts of waste are to be processed these days, it is increasingly preferred to upscale the existing reactors. So far this was not possible with existing reactors; the present device, on the other hand, which comprises a retaining wall, makes it possible to operate large-scale fermentation plants in a safe manner.

The present invention also makes it possible to introduce material to be fermented into the reactor in such a manner that the volume of the reactor is optimally utilised, that is, that the volume of the reactor is filled as well as possible. Without the presence of a retaining wall, a sloping mount of material to be fermented would be formed; the retaining wall, however, makes it possible to pile the material up high as far as the retaining wall, thus providing additional volume of material to be fermented, so that the present invention can be maximally utilised.

In a third embodiment, the present invention relates to a device for carrying out a fermentation process in a reactor, which device comprises a pipe for discharging biogas from the reactor and means for providing access to the reactor for supplying and discharging material to be fermented and fermented material, respectively, wherein the reactor comprises a pipe for supplying a flushing gas to the reactor.

The present invention also relates to a method for carrying out a fermentation process in a reactor, wherein the material to be fermented is moisturised, biogas is produced and the fermented material is removed from the reactor, wherein the reactor is flushed with a flushing gas before the fermented material is removed from the reactor.

According to this embodiment of the present invention, too, the methane gas-containing gas mixture that is present in the reactor is displaced by a flushing gas, so that the risk of explosion is considered to be practically zero. The flushing gas must be an inert gas that cannot form an explosive mixture with the methane gas that is present in the reactor, for example nitrogen, carbon dioxide and carbon monoxide, or mixtures thereof.

Although it has been indicated in the foregoing that the flushing with flushing gas takes place before the fermented material is removed from the reactor, it must be understood that according to the present invention said flushing of the reactor can also take place when a person or persons wish(es) to enter the reactor, for example for inspection or maintenance. Such a situation is to be understood to fall within the scope of the present invention as well.

When a person or persons need(s) to enter the reactor after said flushing with a flushing gas, it is in particular desirable to pass an oxygen-containing mixture, in particular air, through the reactor after said flushing with the flushing gas. In such a situation, the amount of oxygen that is present in the reactor will suffice for said person or persons, so that there is no longer any risk of suffocation.

To ensure that the biogas-containing gas mixture that is present in the reactor is displaced to a sufficient extent, said flushing with a flushing gas must preferably take place with a volume flow of at least five times the volume of the reactor per hour. If a lower volume flow is used when flushing with a flushing gas, there is still a chance that an explosive gas mixture will be present in the reactor, which must absolutely be prevented. The term volume of the reactor is to be understood the net volume, viz. the volume that actually takes part in the flushing process.

Exhaust gases from a combustion engine have proved to be very suitable for use as a flushing gas, which exhaust gases mainly contain carbon dioxide and water. Such a flushing gas is very advantageous from an energetic point of view, since the biogas that is produced in the reactor can be supplied to the combustion engine as a combustion gas, which combustion engine forms part of a so-called thermal power unit, so that the energy that is present in the biogas is usefully reused.

In a special embodiment, the exhaust gases from the combustion engine are preferably passed through a washing unit before they can be supplied to the reactor as a flushing gas. Pre-treatment of the exhaust gases in a so-called scrubber is in particular desirable in order to remove potential ignition sources, such as sparks, as well as environmentally burdening components from the gas. In addition, the hot gases from the combustion engine are cooled in the scrubber.

The flushing of the reactor with a flushing as can take place at any desired moment, but generally it is carried out at a point in time when a person or persons need(s) to enter the reactor (phase 3 of a fermentation process). In such a situation it is desirable that the moisturisation of the material to be fermented is terminated before the flushing of the reactor with a flushing gas is started, after which the material to be fermented can drain out for a few hours, if desired. When said moisturisation is discontinued, the fermentation process will stop, so that the formation of biogas decreases. Once the biogas that is present in the reactor has been displaced by the flushing gas, it is desirable for the flushing gas to be displaced in turn by an oxygen-containing gas mixture, such as air. The gas mixture exiting the reactor is removed therefrom by means of an excess of air and discharged into the atmosphere via a filter unit. According to the present invention, the formation of an explosive gas mixture in the reactor is prevented in this manner.

It is also possible to carry out said flushing during the first phase of the fermentation process, as indicated above, i.e. just after the reactor has been filled, so that the oxygen that is still present will be displaced more rapidly, thus accelerating the fermentation process (phase 2).

The pipe for supplying flushing gas to the reactor serves to prevent the occurrence of explosive gas mixtures in the reactor, which gas mixtures have formed in the course of the fermentation process, in particular when a person or persons need(s) to enter the reactor.

In a special embodiment, the pipe for supplying flushing gas is connected to a pipe through which exhaust gases from a combustion engine are passed, which exhaust gases are used as a flushing gas. Fans, compressors and/or moisturizing installations may be mounted in the aforesaid pipe.

To obtain an advantageous energy household, the pipe for discharging biogas from the reactor is connected to the combustion engine, in which combustion engine the biogas-containing gas mixture from the reactor is combusted so as to generate energy.

Another effect achieved with the present invention is that the occurrence of explosive gas mixtures in a reactor in which a fermentation process takes place is prevented, which occurrence of an explosive gas mixture must be prevented in particular when a person or persons need(s) to enter the reactor, for example for removing the fermented material from the reactor or for subjecting the reactor to an inspection. The present step of flushing the reactor with the flushing gas will prevent the occurrence of explosive gas mixtures, so that a person or persons can enter the reactor under safe conditions.

The various embodiments of the present invention will be explained in more detail hereinafter with reference to the associated drawings, in which:
Figure 1 is a perspective view of the first embodiment of the present invention.
Figure 2 is a sectional view of the first embodiment of the present invention, seen from above in the direction of the arrow that is shown in figure 1.
Figure 3 is a sectional view of the first embodiment of the present invention, seen from aside in the direction of the arrow that is shown in figure 1.
Figure 4 is a perspective view of the second embodiment of the present invention.
Figure 5 is a sectional view of the second embodiment of the present invention, seen from above in the direction of the arrow that is shown in figure 4.
Figure 6 is a sectional view of the second embodiment of the present invention, seen from aside in the direction of the arrow that is shown in figure 4.
Figure 7 is a top plan view (figure 7a) and a side view (figure 7b) of a U-shaped section according to a preferred embodiment of the present invention.
Figure 8 shows an embodiment of a wall element according to the present invention.
Figure 9 is a cross-sectional view of another embodiment of the present invention.
Figure 10 schematically shows the principle of the flow of gases according to one embodiment of the present invention.
In the figures, like parts are indicated by the same numerals.

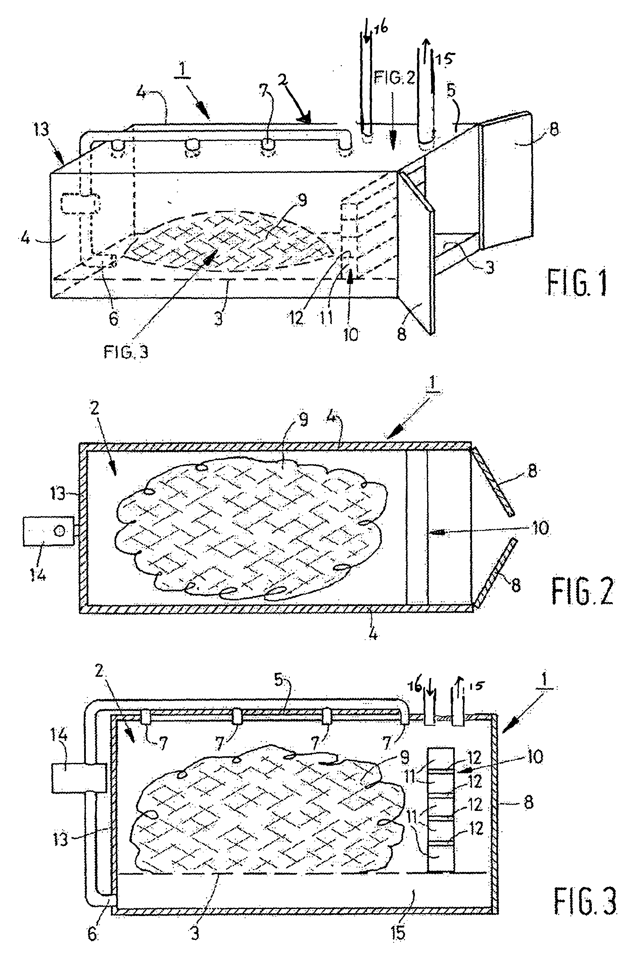

Figure 1 shows a device 1 provided with a reactor 2 comprising a liquid-permeable floor 3, two substantially parallel side walls 4 extending perpendicularly to the floor 3, a rear wall 13 and a ceiling part 5 disposed opposite the floor 3, which joins said side walls 4 and said rear wall 13. The device 1 further comprises means 8 for providing access to the reactor 2. Underneath the surface of the floor 3, the reactor 2 is provided with a pipe 6 for discharging liquid from the reactor 2. Said pipe 6 may be connected to means 7 for moisturising the material 9 to be fermented, which material 9 is supported on the floor 3. In addition to that, a retaining wall 10 is shown, which is preferably built up of individual wall elements 11. Passages 12 may be present between the individual wall elements, through which passages liquid can flow. Furthermore, the device 1 is preferably provided with a pipe 15 at an upper side, for example in the ceiling part 5 or at the upper side of the side walls 4 or the rear wall 13, which pipe functions to discharge biogas from the reactor 2, and with a pipe 16 for supplying a flushing gas to the reactor 2, which pipe is preferably also disposed at the upper side of the reactor 2, in particular above the material 9 that is to be fermented or that has been fermented.

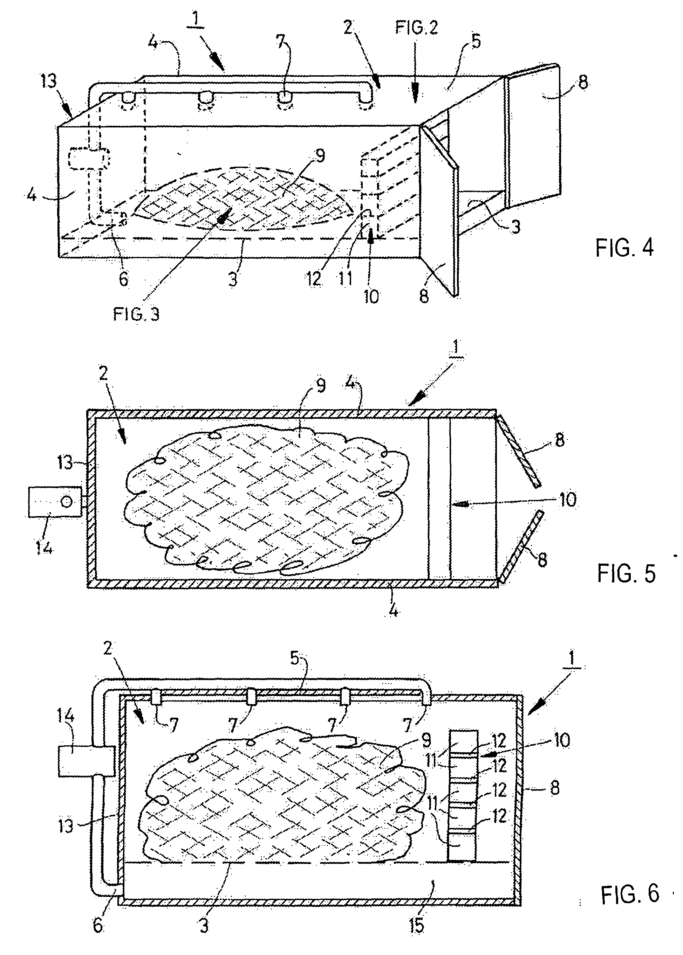

Figure 2 is a top plan view of the device 1, which shows clearly that the retaining wall 10 is enclosed by the side walls 4. The retaining wall 10 will not be present in the reactor prior to the fermentation process, and the reactor 2 can be filled with the material 9 to be fermented, for example by means of a shovel (not shown). The reactor 2 can be entered via the means 8 for providing access to the reactor 2, which means are here in the form of two swing doors. It will be apparent, however, that also other means 8 for providing access to the reactor 2 may be used. In a preferred embodiment, the material 9 to be fermented will be deposited on the floor 3 of the reactor 2 by means of a loading vehicle (not shown), such as a shovel. Such a loading vehicle can enter the reactor 2 through the doors 8 and deposit the material 9 to be fermented on the floor 3, which is liquid-permeable. The floor 3 may be a grid, after example, or a floor provided with a number of holes, so that the liquid can flow out of the fermented material 9 into a space underneath the floor 3 (as shown in figure 3).

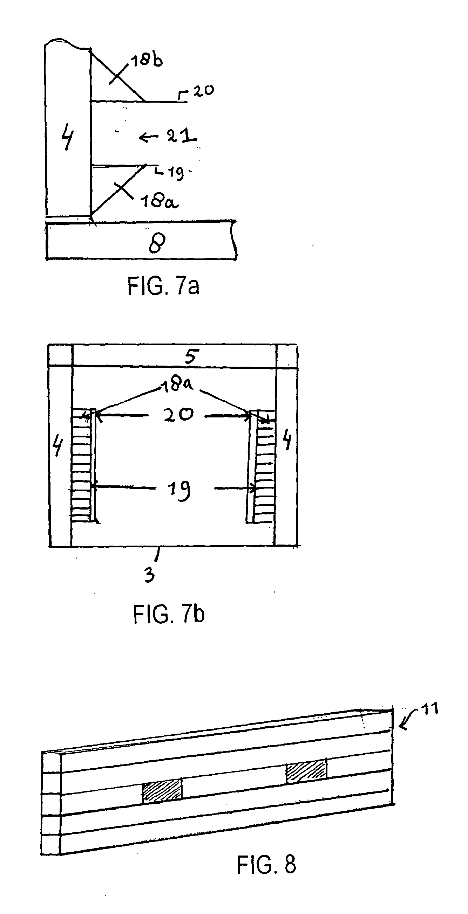

In figure 3, the material 9 to be fermented is shown to be present on the floor 3 of the reactor 2. After the reactor 2 has been filled with the material 9 to be fermented, a retaining wall 10 can be installed. It is preferable to install the retaining wall 10 by stacking up individual wall elements 11. Such wall elements 11 can be placed into a U-shaped section (see figure 7), for example, which section is mounted to the side walls 4 of the reactor 2. The use of such a U-shaped section enables an easy installation and removal of the wall elements 11 prior to and after the fermentation process. Furthermore, a very stable retaining wall 10 built up of stacked-together wall elements 11 is obtained when such a section is used. The wall elements 11 can be installed by means of a loading vehicle, for example, such as the aforesaid shovel used for filling the reactor 2. The retaining wall 10 is enclosed by the side walls 4 (see figure 2), thus forming a closed space enclosed by the rear wall 13 , the side walls 4, the retaining wall 10 and the floor 3, in which space the material 9 to be fermented is present.
Figures 4-6 show the second embodiment of the present invention, in which like parts are indicated by the same numerals. Reference is made in this connection to the above description of the embodiment according to figures 1-3.
Figure 7 shows a top plan view (figure 7a) and a side view (figure 7b) of the U-shaped section according to a preferred embodiment of the present invention. Figure 7a shows part of a side wall 4, the means 8 and the U-shaped section composed of two stiffening ribs 18a and 18b, which are fixed side-by-side to the side wall 4, near the means 8. The stiffening rib 18a is provided with a front portion 19 of a guide 21, and the stiffening rib 18b is provided with a rear portion 20 of a guide 22. Figure 7b is a front view of the same the U-shaped section. The figure shows two side walls 4, a floor 3 and a ceiling part 5, as well as stiffening ribs 18a and a front portion 19 and a rear portion 20 of the guide. The U-shaped section provided with guides 21 that is formed on both side walls 4 in this manner functions to receive the wall elements 11 of the retaining wall 10. Thus, one lateral end of a wall element 11 is placed into one of the guides 21 and the other lateral end of the wall element 11 is placed into the other guide 21.
Figure 8 shows a preferred embodiment of a wall element 11 of the retaining wall 10 according to the present invention. The present retaining wall 10 consists of a number of wall elements 11 (for example four), one of which is shown in figure 8. The wall element 11 that is shown in figure 8 consists of five beams (for example 0.2x0.2x6m), which are connected together. One or more openings (two in the illustrated embodiment) may be present in one or more of the beams (in the middle beam in the illustrated embodiment), which makes it easier to place the wall element 11, for example by means of a forklift truck.

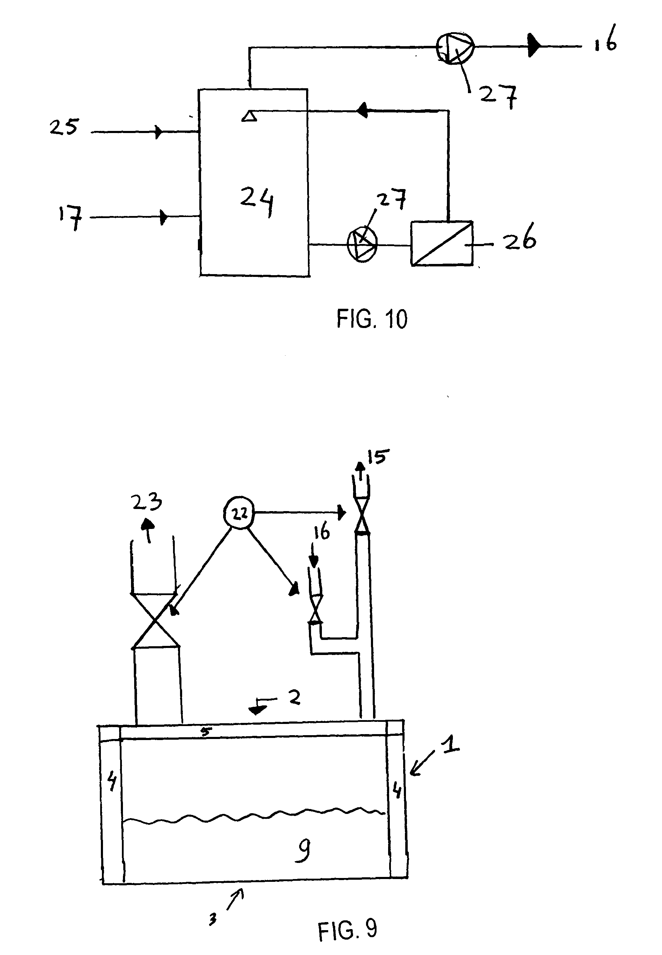

Figure 9 is a cross-sectional view of the present device 1, showing the reactor 2 comprising a floor 3, two side walls 4 and a ceiling part 5. A pipe 15 for discharging biogas is provided in the ceiling part 5, and a pipe 16 for supplying flushing gas is connected to said pipe 15 outside the reactor 2. This arrangement is an alternative to the embodiment that is shown in figure 1, therefore, in which the pipe 16 is directly connected to the reactor 2. The arrangement that is shown in figure 9 leads to a reactor 2 with a minimum number of connections, which simplifies the production of the reactor 2. Figure 9 furthermore shows a number of valves 22, by means of which the various pipes can be shut off and opened. In addition to that, the reactor 2 may be provided with a ventilating channel 23.
Figure 10 schematically shows the principle of an embodiment of the present invention, showing the pipe 16. The reactor 2 and the pipe 15 are not shown. The device according to the present embodiment furthermore comprises a pipe 17, through which exhaust gases from a combustion engine are passed and which is connected to the pipe 16 via a cooling tower 24 and a valve 27. Furthermore, a pipe 25 for supplying air (preferably outside air) is connected to the cooling tower 24. The cooling tower 24 is cooled by means of the cooling water circuit 26.
The capacity of the present device is a few hundred cubic metres, for example, and the present invention will have a width and a height of 6 m each and a length of 20 m, for example. Connections for the pipe 14 for discharging biogas from the reactor 2 and means 7 for moisturising the material 9 to be fermented in the reactor 2 may be present in the ceiling part 5. The side walls 4, the rear wall 3 and the ceiling part of 5 are preferably liquid-tight and/or airtight. Furthermore it is preferable if the means 8 for providing access to the reactor 2 are liquid-tight and/or airtight in closed condition. Another advantage of using the retaining wall 10 is that it need not be liquid-tight and/or airtight, and that less stringent requirements may be made as regards the strength of the means 8 when using the retaining wall 10. Thus it is no longer necessary to combine the three requirements (robustness, liquid-tightness and airtightness) into a single structural component, but to distribute them over two components (the means 8 and the retaining wall 10).

## Claims

1. A device (1) for carrying out a fermentation process in a reactor (2), which reactor (2) comprises a liquid-permeable floor (3), two substantially parallel side walls (4) extending perpendicularly to the floor (3), a rear wall (13), and a ceiling part (5) disposed opposite the floor (3), which joins said side walls (4) and said rear wall (13), which device (1) further comprises a pipe (6) for discharging liquid from the reactor (2), which pipe (6) is disposed underneath the floor (3), and which device (1) further comprises means (7) for moisturising the material to be fermented (9) that is present in the reactor (2) as well as means (8) for providing access to the reactor (2) for the purpose of supplying and discharging material (9) to be fermented and fermented material (9), respectively, and which device (1) furthermore comprises a pipe (15) for discharging biogas from the reactor (2), **characterized in that** the reactor (2) comprises a retaining wall (10), which retaining wall (10) is disposed between the means (8) for providing access to the reactor (2) and the material (9) to be fermented that is present on the floor (3), wherein the retaining wall (10) is supported on the floor (3), being enclosed between the two side walls (4), and wherein the reactor (2) is provided with a pipe (16) for supplying a flushing gas to the reactor (2).

2. A device (1) for carrying out a fermentation process in a reactor (2), which reactor (2) comprises a liquid-permeable floor (3), two substantially parallel side walls (4) extending perpendicularly to the floor (3), a rear wall (13), and a ceiling part (5) disposed opposite the floor (3), which joins said side walls (4) and said rear wall (13), which device (1) further comprises a pipe (6) for discharging liquid from the reactor (2), which pipe (6) is disposed underneath the floor (3), and which device (1) furthermore comprises means (7) for moisturising the material to be fermented (9) that is present in the reactor (2) as well as means (8) for providing access to the reactor (2) for the purpose of supplying and discharging material (9) to be fermented and fermented material (9), respectively, **characterized in that** the reactor (2) comprises a retaining wall (10), which retaining wall (10) is disposed between the means (8) for providing access to the reactor (2) and the material (9) to be fermented that is present on the floor (3), wherein the retaining wall (10) is supported on the floor (3), being enclosed between the two side walls (4).

3. A device according to either one or both of the claims 1-2,
**characterized in that** the retaining wall (10) consists of individual wall elements (11) to be placed one on top of the other.

4. A device according to claim 3, **characterized in that** one or more passages (12) through which liquid can flow are present between the individual wall elements (11) of the retaining wall (10).

5. A device according to any one or more of the claims 1-4, **characterized in that** the liquid-permeable floor (3) extends as far as the means (8) for providing access to the reactor (2).

6. A device (1) for carrying out a fermentation process in a reactor (2), which device (1) comprises a pipe (15) for discharging biogas from the reactor (2) and means (8) for providing access to the reactor (2) for supplying and discharging material (9) to be fermented and fermented material (9), respectively, **characterized in that** the reactor (2) comprises a pipe (16) for supplying a flushing gas to the reactor (2).

7. A device (1) according to claim 6, **characterized in that** the pipe (16) for supplying a flushing gas is connected to a pipe (17) through which exhaust gases from a combustion engine are carried.

8. A device (1) according to either one or both of the claims 6-7, **characterized in that** the pipe (15) for discharging biogas from the reactor (2) serves as a supply pipe to the combustion engine.

9. A method for carrying out a fermentation process in a reactor (2), wherein the material (9) to be fermented is moisturised, biogas is produced and the fermented material (9) is removed from the reactor (2), **characterized in that** the reactor (2) is flushed with a flushing gas before the fermented material (9) is removed from the reactor (2).

10. A method according to claim 9, **characterized in that** air is passed through the reactor (2) after said flushing with a flushing gas.

11. A method according to claim 10, **characterized in that** said flushing with a flushing gas takes place with a volume flow of at least five times the volume of the reactor (2) per hour.

12. A method according to any one or more of the claims 9-11, **characterized in that** exhaust gases from a combustion engine are used as the flushing gas.

13. A method according to claim 12, **characterized in that** the biogas that is produced in the reactor (2) is supplied to the combustion engine.

14. A method according to any one or more of the claims 12-13, **characterized in that** the exhaust gases from the combustion engine are passed through a washing unit before being supplied to the reactor (2) as a flushing gas.

15. A method according to any one or more of the claims 9-14, **characterized in that** the flushing gas exiting the reactor (2) is passed through a filter unit.

16. A method according to any one or more of the claims 9-15, **characterized in that** the moisturisation of the fermented material in the reactor (2) is terminated before the flushing of the reactor (2) with a flushing gas is started.
